(19) Europäisches Patentamt
European Patent Office
Office européen des brevets

(11) Veröffentlichungsnummer : **0 361 168 B1**

(12) # EUROPÄISCHE PATENTSCHRIFT

(45) Veröffentlichungstag der Patentschrift :
**25.11.92 Patentblatt 92/48**

(51) Int. Cl.$^5$ : **C07D 307/64,** C07D 333/34, A23L 1/226

(21) Anmeldenummer : **89116587.0**

(22) Anmeldetag : **08.09.89**

(54) **Halbmercaptale, Verfahren zu ihrer Herstellung und ihre Verwendung.**

(30) Priorität : **21.09.88 DE 3831981**

(43) Veröffentlichungstag der Anmeldung :
**04.04.90 Patentblatt 90/14**

(45) Bekanntmachung des Hinweises auf die Patenterteilung :
**25.11.92 Patentblatt 92/48**

(84) Benannte Vertragsstaaten :
**CH DE FR GB IT LI NL**

(56) Entgegenhaltungen :
**EP-A- 0 043 486**
**US-A- 3 653 920**

(73) Patentinhaber : **HAARMANN & REIMER GMBH**
**Postfach 138**
**W-3450 Holzminden (DE)**

(72) Erfinder : **Brüning, Jürgen, Dr.**
**Sparenberg 39**
**W-3450 Holzminden (DE)**
Erfinder : **Emberger, Roland, Dr.**
**Bärenfang 4**
**W-3450 Holzminden (DE)**
Erfinder : **Güntert, Matthias, Dr.**
**Vogelsang 4**
**W-3450 Holzminden (DE)**
Erfinder : **Hopp, Rudolf, Dr.**
**Auf dem Gehrenkamp 28**
**W-3450 Holzminden (DE)**
Erfinder : **Köpsel, Manfred, Dr.**
**Schinkelstrasse 1**
**W-3450 Holzminden (DE)**
Erfinder : **Sand, Theodor, Dr.**
**Vogelsang 3**
**W-3450 Holzminden (DE)**
Erfinder : **Werkhoff, Peter, Dr.**
**Sieplerstrasse 24**
**W-3470 Höxter 1 (DE)**

(74) Vertreter : **Müller, Gerhard, Dr. et al**
**BAYER AG Konzernverwaltung RP**
**Patentabteilung**
**W-5090 Leverkusen 1 Bayerwerk (DE)**

**EP 0 361 168 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue Halbmercaptale, ein Verfahren zu ihrer Herstellung und ihre Verwendung als Aromastoffe.

Es wurden neue Halbmercaptale der Formel

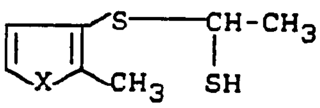

(I),

in der

X für ein Sauerstoff- oder Schwefelatom steht,

gefunden, die wertvolle organoleptische Eigenschaften aufweisen.

Die erfindungsgemäßen Halbmercaptale der Formel (I) werden durch Kondensation von Thiolen der Formel

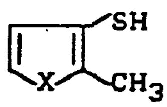

(II),

in der

X die unter Formel (I) angegebene Bedeutung hat,

mit Acetaldehyd und Behandlung der bei der Kondensation entstehenden Verbindungen der Formel

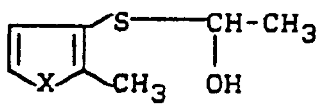

(III),

in der

X die unter Formel (I) angegebene Bedeutung hat,

mit Schwefelwasserstoff erhalten.

Die Erfindung betrifft daher auch ein Verfahren zur Herstellung von Halbmercaptalen der Formel (I), das dadurch gekennzeichnet ist, daß man Thiole der Formel (II) mit Acetaldehyd in Gegenwart von Schwefelwasserstoff in wäßriger Lösung bei Temperaturen von 0°C bis 10°C in Gegenwart einer organischen Säure, vorzugsweise Essigsäure, kondensiert. Beide Verfahrensstufen, Kondensation und Überführen der Hydroxygruppe des Kondensationsproduktes in die SH-Gruppe, lassen sich zu einer 1-Stufenreaktionverbindung verbinden, in dem man die Kondensation unmittelbar in Gegenwart von Schwefelwasserstoff vornimmt.

Die als Ausgangsverbindungen benötigten Thiole der Formel (II) sind bekannt (siehe DE-OS 24 58 609).

Die erfindungsgemäßen Verbindungen sind wertvolle Aromastoffe, die sich durch sehr niedrige Geschmacksschwellenwerte auszeichnen. Die Geschmacksbeschreibungen lauten:

1-(2-Methyl-3-furylthio)-ethanthiol:

röstig, fleischig, Brühe, Gemüse

1-(2-Methyl-3-thienylthio)-ethanthiol:

Brühe, fleischig, Schweinefleisch, schwefelig, hefig

Mit ihrem spezifischen Geschmack in Richtung Fleisch wirken die erfindungsgemäßen Verbindungen der Formel (I) in Fleischaromakompositionen geschmacksverstärkend und -abrundend. Aber auch in anderen Aromakompositionen, z.B. Nußaromen, bewirken die erfindungsgemäßen Verbindungen eine Abrundung des Aromas und eine Erhöhung der Geschmacksfülle.

Die unter Verwendung der erfindungsgemäßen Verbindungen hergestellten Aromakompositionen können im gesamten Nahrungsmittel- und Genußmittelbereich, sowie in Tierfutter eingesetzt werden. Insbesondere sind sie geeignet für Fettmassen, Backwaren, Extrusionsprodukte, Fertiggerichte, Fleisch- und Wurstprodukte, Suppen, Soßen, Gemüsekonserven und alle Arten von industriell gefertigtem Tierfutter.

Die erfindungsgemäßen neuen Halbmercaptale werden in Mengen von 5 ppt bis 1 %, vorzugsweise 100 ppt bis 100 ppm, bezogen auf das verzehrfertige Nahrungsmittel verwendet.

Bei den in den Beispielen verwendeten %-Angaben handelt es sich um Gewichts-%.

Beispiel 1

Eine bei 0°C mit Schwefelwasserstoff gesättigte Lösung von 4 g (0,1 Mol) Natriumhydroxid in 36 ml Wasser wird mit 30 ml Methylenchlorid und 0,5 ml Essigsäure versetzt. Zur Mischung werden unter Rühren 4,4 g (0,1 Mol) Acetaldehyd und 13 g (0,1 Mol) 2-Methyl-3-thienylthiol zugetropft. Der pH-Wert des Reaktionsgemisches wird durch Zugabe von etwa 10 g Essigsäure auf 5 bis 6 gehalten. Während des Zutropfens und der 2-stündigen Nachreaktion bei Raumtemperatur wird Schwefelwasserstoff eingeleitet um die Sättigung der Lösung an Schwefelwasserstoff aufrecht zu erhalten. Nach 12-stündigem Aufbewahren bei Raumtemperatur wird das Reaktionsgemisch durch Zugabe von Natriumhydroxid alkalisch gemacht und die organische Phase abgetrennt und verworfen. Die wäßrige Phase wird mit verdünnter Salzsäure angesäuert und mit Ether extrahiert. Die vereinigten Etherextrakte werden in üblicher Weise aufgearbeitet. Es werden 3 g Rohprodukt erhalten; diese liefern nach der Destillation bei 71°C/0,3 mbar 0,3 g 1-(2-Methyl-3-thienylthio)-ethanthiol.

Eine durch präparative Gaschromatographie gereinigte Probe der Verbindung wurde durch ihre IR-, NMR- und Massenspektren identifiziert.

Beispiel 2

Es wurde wie in Beispiel 1 beschrieben gearbeitet nur wurde anstelle von 2-Methyl-3-thienylthiol eine äquivalente Menge (0,1 Mol) 2-Methyl-3-furylthiol eingesetzt.

Es wurden 0,5 g 1-(2-Methyl-3-furylthio)-ethanthiol erhalten. Eine durch präparative Gaschromatographie gereinigte Probe der Verbindung wurde durch ihre IR-, NMR- und Massenspektren identifiziert.

Beispiel 3

Eine Fleischaromakomposition wird durch Mischen folgender Bestandteile hergestellt:

| | |
|---|---|
| 50:50-Mischung von Na-Inosinat und Na-Guanilat | 1 |
| Mononatriumglutamat | 19 |
| Milchsäure, sprühgetrocknet | 30 |
| Pflanzenproteinhydrolysat (Type RFB der FIS) | 350 |
| Süßmolkepulver | 100 |
| Speisesalz | 500 |
| | 1000 |

Eine 1 %ige wäßrige Lösung dieser Komposition dient als Kontrollprobe.

Wird die Kontrollprobe mit 500 ppt 1-(2-Methyl-3-furylthio)-ethanthiol versetzt, so wird der Geschmack der wäßrigen Lösung von einer Testgruppe im Vergleich zur Kontrollprobe als deutlich fleischiger beschrieben. Der gleiche Effekt wird durch die Zugabe von 2 ppb 1-(2-Methyl-3-thienylthio)-ethanthiol zur Kontrollprobe erreicht.

## Patentansprüche

1. Halbmercaptale der Formel

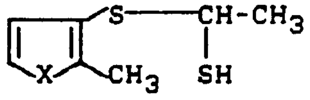

(I),

in der
X ein Sauerstoff- oder ein Schwefelatom darstellt.

**2.** Verfahren zur Herstellung von Halbmercaptalen der Formel

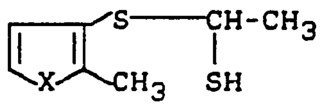

(I),

in der
X ein Sauerstoff- oder ein Schwefelatom darstellt,
dadurch gekennzeichnet, daß man Thiole der Formel

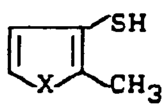

(II),

in der
X die unter Formel (I) angegebene Bedeutung hat,
mit Acetaldehyd in Gegenwart von $H_2S$ kondensiert.

**3.** Verwendung der Halbmercaptale nach Anspruch 1 als Aromastoffe.

## Claims

**1.** Hemimercaptals of the formula

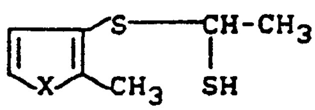

(I)

in which
X represents an oxygen or a sulphur atom.

**2.** Process for the preparation of hemimercaptals of the formula

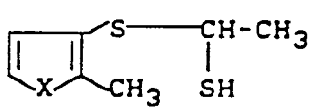

(I)

in which
X represents an oxygen or a sulphur atom,
characterized in that thiols of the formula

(II)

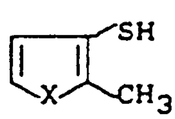

in which

X has the meaning indicated under formula (I), are condensed with acetaldehyde in the presence of $H_2S$.

3. Use of hemimercaptals according to Claim 1 as flavourings.

**Revendications**

1. Hémimercaptals de formule

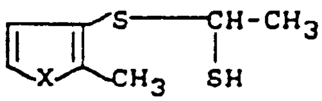

(I)

dans laquelle
X représente un atome d'oxygène ou de soufre.

2. Procédé de préparation des hémimercaptals de formule:

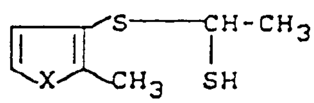

(I)

dans laquelle
X représente un atome d'oxygène ou de soufre,
caractérisé en ce que l'on condense avec l'acétaldéhyde des thiols de formule

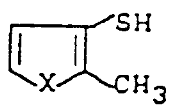

(II)

dans laquelle
X a la signification donnée au sujet de la formule (I),
en présence de $H_2S$.

3. Utilisation des hémimercaptals selon la revendication 1 comme aromatisants.